## Europäisches Patentamt
## European Patent Office
## Office européen des brevets

(11) Numéro de publication: **0 038 738**
**A1**

(12) **DEMANDE DE BREVET EUROPEEN**

(21) Numéro de dépôt: **81400562.5**

(22) Date de dépôt: **09.04.81**

(51) Int. Cl.³: **G 01 N 33/80**

(30) Priorité: **21.04.80 FR 8008893**

(43) Date de publication de la demande:
**28.10.81 Bulletin 81/43**

(84) Etats contractants désignés:
**BE CH DE GB IT LI LU NL SE**

(71) Demandeur: **Fella, Christian Paul**
**26, Boulevard de Cimiez**
**F-06000 Nice(FR)**

(71) Demandeur: **Hammou, Jean-Claude**
**32, Avenue du Dauphiné**
**F-06000 Nice(FR)**

(72) Inventeur: **Fella, Christian Paul**
**26, Boulevard de Cimiez**
**F-06000 Nice(FR)**

(72) Inventeur: **Hammou, Jean-Claude**
**32, Avenue du Dauphiné**
**F-06000 Nice(FR)**

(74) Mandataire: **Barnay, André François et al,**
**Cabinet Barnay 80 rue Saint-Lazare**
**F-75009 Paris(FR)**

(54) Procédé de détection de la présence d'antigènes d'un groupe sanguin dans une coupe histologique, et produits pour mettre en oeuvre ce procédé.

(57) L'invention permet de détecter facilement et avec précision la présence d'antigènes d'un groupe sanguin A, B, O ou AB dans une coupe histologique.

On expose la coupe histologique à un antisérum monospécifique de l'antigène A, B ou H (O) ou AB. Les anticorps (2) de l'antisérum sont marqués, avant ou après cette exposition, par des éléments marqueurs (4). Après fixation des anticorps aux cellules porteuses d'antigène (1) et lavage de la coupe histologique, on peut mettre en évidence la présence des antigènes par un examen optique.

Les produits pour mettre en oeuvre l'invention sont des antisérums monospécifiques de l'antigène A, B, O ou AB, marqués ou non par une substance fluorescente. Si le marquage n'est pas fait à l'avance, les produits comportent des récipients séparés. L'un contient ledit antisérum et l'autre un sérum anti-immunoglobuline marqué.

Application à l'évaluation pronostique des tumeurs malignes.

Procédé de détection de la présence d'antigènes d'un groupe sanguin dans une coupe histologique, et produits pour mettre en oeuvre ce procédé.

La présente invention concerne la mise en évidence, au niveau des tissus humains, d'antigènes d'un groupe sanguin A, B, O ou AB.

Jusqu'à présent, la recherche de la présence de ces antigènes a été effectuée au niveau des tissus normaux grâce à une technique utilisée depuis 1956. Cette technique a révélé que l'antigène correspondant au groupe sanguin d'un individu est toujours présent à la surface des cellules qui composent ces tissus.

La technique utilisée dans l'art antérieur utilise le marquage des antigènes par des globules rouges. Cette méthode, peu maniable, ne convient pas pour la pratique courante, pour les raisons suivantes : manque de rapidité, fiabilité incertaine (appréciation à plus ou moins 30%), donc difficulté de quantification, et reproductibilité très restreinte du fait de la variété du marqueur utilisé et des conséquences de sa fragilité (phénomène d'hémolyse, fausse agglutination, mauvaise conservation, impossibilité de congélation).

Tous ces facteurs, et notamment le manque de fiabilité du marqueur, limitent la diffusion de cette méthode connue.

Or, des travaux ont montré que la présence ou l'absence d'un antigène du type précité constitue un critère d'évaluation pronostique des tumeurs malignes. C'est ainsi que la méthode connue sus-mentionnée a été récemment utilisée pour rechercher à la surface des tumeurs malignes (sein, estomac, côlon, col utérin, etc...) l'existence des antigènes de groupes sanguins. En particulier, les derniers travaux ayant porté sur les tumeurs de vessie ont donné par cette technique des résultats intéressants en ce qui concerne l'évaluation pronostique pour ce type tumoral.

La présente invention a pour but de parvenir à un procédé de détection de la présence d'antigènes d'un groupe sanguin A, B, O ou AB dans une coupe histologique, ce procédé étant exempt des inconvénients de l'art antérieur et pouvant être mis en oeuvre non pas seulement par un chercheur mais aussi par un laboratoire médical standard ou par un praticien ayant besoin d'un résultat immédiat et fiable, permettant de ce fait une thérapeutique appropriée.

Selon l'invention, ce but est atteint par le fait qu'on expose la coupe histologique à un antisérum qui est monospécifique de l'antigène A ou B ou H (O) ou AB recherché, et, au moyen d'une substance fluorescente, on marque - directement ou indirectement - l'anticorps contenu dans cet antisérum, et par le fait qu'après fixation et marquage, ou marquage et fixation, de cet anticorps sur les antigènes de la coupe histologique, on lave cette dernière et on décèle optiquement la présence éventuelle de l'agent marqueur sur cette coupe histologique, une telle présence témoignant que ladite coupe histologique comporte des antigènes du groupe considéré, puisque l'anticorps a été fixé.

Ce procédé admet plusieurs variantes :

Dans une première variante, le marquage de l'antisérum, c'est-à-dire de l'anticorps qu'il contient, est effectué avant l'exposition de la coupe histologique à l'antisérum. Dans ce cas, l'agent marqueur avec lequel l'antisérum est marqué peut être une substance fluorescente, par exemple la fluorescéine (isothiocyanate de fluorescéine par exemple) ou la rhodamine, et c'est alors par un examen en lumière ultraviolette que l'on décèle la présence éventuelle de l'agent marqueur sur la coupe histologique.

Dans une deuxième variante, c'est après avoir exposé la coupe histologique à l'antisérum que l'on marque l'anticorps fixé par elle. Ce marquage de l'anticorps fixé peut alors être effectué avec un autre sérum, à savoir un sérum anti-immunoglobuline humaine ou non, marqué, par exemple, par un agent fluorescent tel que fluorescéine ou rhodamine, l'examen optique de la coupe histologique étant ensuite effectué en lumière ultraviolette.

Le marquage d'un antisérum est une opération bien connue de l'homme de l'art opérant dans les laboratoires de recherche et n'a donc pas à être décrit. Il n'a jusqu'à présent jamais été proposé, ni même suggéré, de préparer systématiquement à l'avance et de vendre, à des fins de diagnostic standard courant, des antisérums marqués ou marquables tels que ceux mentionnés dans la présente description en vue de mettre en oeuvre l'invention.

Le terme "antisérum" désigne, d'une façon générale, des anticorps qui sont utilisés en suspension. Leur origine peut être quelconque (homme, animal, culture de cellules, etc.).

L'invention a également pour objet des produits pour mettre en oeuvre le procédé selon l'invention.

Un tel produit convenant pour la mise en oeuvre du procédé dans la première variante est un antisérum monospécifique de l'un des groupes A, B, O ou AB, cet antisérum étant marqué par une substance fluorescente, telle que fluorescéine ou rhodamine (par exemple).

Pour mettre en oeuvre la deuxième variante du procédé, l'invention prévoit un antisérum monospécifique A, B, O ou AB non marqué, contenu dans un premier récipient, et un sérum anti-immunoglobuline humaine ou non, contenu dans un deuxième récipient et marqué par une substance fluorescente telle que, par exemple, isothiocyanate de fluorescéine ou rhodamine.

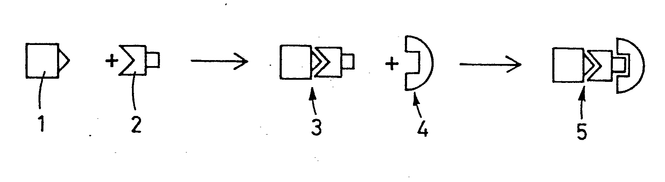

La figure unique du dessin annexé illustre la réaction mise en oeuvre dans le procédé.

Dans le cas du marquage indirect, c'est-à-dire dans le cas de la deuxième variante, la référence 1 désigne une cellule avec son antigène, tandis que la référence 2 désigne un anticorps spécifique contenu dans l'antisérum non marqué mis en présence de cette cellule 1. La référence 3 désigne le corps résultant de la fixation de l'anticorps 2 sur la cellule 1. La référence 4 désigne un marqueur, par exemple une anti-immunoglobuline humaine ou non, marquée mise en présence du corps 3. La référence 5 désigne le corps complexe résultant de la fixation de l'anti-immunoglobuline marquée sur le corps 3. On comprend que si, après élimination complète (par lavage) de tous les éléments 2 et 4 non fixés, l'examen optique, par une méthode connue (par exemple au microscope), révèle la présence d'éléments 4 fixés, cela traduit la présence de l'antigène 1.

Si l'on opère selon la première variante (donc au moyen d'un antisérum déjà marqué), c'est alors un corps composé, formé par la fixation d'un marqueur 4 à un anticorps 2,que l'on met en présence de la cellule avec antigène 1, pour obtenir finalement le corps complexe 5 identifiable optiquement.

Les modalités d'exécution sont particulièrement simples et à la portée de tout laboratoire courant.

On opère avantageusement à partir de préparations histologiques déparaffinées non colorées, confectionnées selon les techniques habituelles déjà connues.

Les préparations histologiques déparaffinées sont fixées dans un bain d'acétone pendant 15 minutes, après quoi on les lave, de préférence dans trois bains différents, avantageusement avec une solution tampon à pH 7,3 (un tampon phosphate commercialisé sous le nom de P.B.S. convient parfaitement). La durée totale du lavage est de l'ordre de 15 minutes. Après séchage des lames (préparations histologiques), on les place dans une boîte métallique, à plat sur des porte-objets et on les recouvre de l'antisérum correspondant. La durée de cette exposition à l'antisérum peut atteindre 30 minutes, voire davantage, sans que cela nuise à la réaction.

Si l'antisérum appliqué est un antisérum déjà marqué par une substance fluorescente, on ajoute de préférence, par exemple au début de l'exposition, quelques gouttes de solution au 1/1000 de bleu Evans (colorant histologique). Cette addition avantageuse peut aussi être faite à l'avance. Le colorant histologique a pour effet d'accroître le contraste ce qui facilite l'examen au microscope.

Si l'antisérum appliqué n'est pas un antisérum déjà marqué, on applique, après lavage succédant à l'exposition, le sérum anti-immunoglobuline humaine ou non, marqué, ou plus avantageusement un sérum anti-immunoglobuline de spécificité M, et l'on procède à une deuxième exposition.

Les préparations sont ensuite lavées, de préférence dans plusieurs bains successifs de solution tempon à pH 7,3 (5 minutes à chaque fois). Après cela on sèche les préparations histologiques et l'on monte les coupes de préférence

5

avec une goutte de glycérol placée sur une lamelle qui est collée sur la préparation.

On procède ensuite à la lecture au microscope sous éclairage correspondant au type de marqueur : éclairage UV et microscope à fluorescence avec filtres appropriés.

Bien entendu, ce mode opératoire n'est pas limitatif et certaines variantes pourraient être envisagées à son égard sans pour autant sortir du cadre de l'invention.

**0038738**

REVENDICATIONS

1.- Procédé de détection de la présence d'antigènes d'un groupe sanguin A, B, O ou AB dans une coupe histologique, caractérisé par le fait que l'on expose la coupe histologique à un antisérum qui est monospécifique de l'antigène A, ou B ou H (O) ou AB recherché, et l'on marque avec une substance fluorescente - cela directement ou indirectement - l'anticorps contenu dans cet antisérum, et par le fait qu'après fixation et marquage, ou marquage et fixation, de cet anticorps sur les antigènes de la coupe histologique, on lave cette dernière et on décèle optiquement la présence éventuelle de l'agent marqueur sur cette coupe histologique, une telle présence témoignant que ladite coupe histologique comporte des antigènes du groupe considéré puisque l'anticorps a été fixé.

2.- Procédé selon la revendication 1, caractérisé par le fait que le marquage de l'antisérum, c'est-à-dire de l'anticorps qu'il contient, est effectué avant l'exposition de la coupe histologique à cet antisérum.

3.- Procédé selon la revendication 2, caractérisé par le fait que l'agent marqueur avec lequel l'antisérum est marqué est la fluorescéine ou la rhodamine ou équivalent, et par le fait que c'est par un examen en lumière ultraviolette que l'on décèle la présence éventuelle de l'agent marqueur sur la coupe histologique.

4.- Procédé selon la revendication 1, caractérisé par le fait que c'est après avoir exposé la coupe histologique à l'antisérum que l'on marque l'anticorps fixé par elle.

5.- Procédé selon la revendication 4, caractérisé par le fait que l'on marque l'anticorps avec un autre sérum, à savoir un sérum anti-immunoglobuline marqué.

6.- Procédé selon la revendication 5, caractérisé par le fait que le sérum anti-immunoglobuline est marqué par un agent fluorescent, par exemple la fluorescéine ou la rhodamine, et par le fait que c'est par un examen en lumière ultraviolette que l'on décèle la présence éventuelle de l'agent marqueur sur la coupe histologique.

7.- Produit pour mettre en oeuvre le procédé selon l'une quelconque des revendications 1, 2 et 3, caractérisé par un antisérum monospécifique de l'un des groupes A, B, O ou AB, cet antisérum étant marqué.

8.- Produit selon la revendication 7, caractérisé par le fait que l'antisérum est marqué par une substance fluorescente telle que par exemple la fluorescéine ou la rhodamine.

9.- Produits pour mettre en oeuvre le procédé selon l'une quelconque des revendications 1, 4, 5 et 6, caractérisés par un antisérum monospécifique A, B, O ou AB non marqué contenu dans un premier récipient, et par un sérum anti-immunoglobuline contenu dans un deuxième récipient et marqué.

10.- Produits selon la revendication 9, caractérisés par le fait que le sérum anti-immunoglobuline est marqué par une substance fluorescente telle que, par exemple, la fluorescéine ou la rhodamine.

0038738

## 0038738
Numéro de la demande

EP 81 40 0562

**Office européen des brevets**

# RAPPORT DE RECHERCHE EUROPEENNE

| | DOCUMENTS CONSIDERES COMME PERTINENTS | | CLASSEMENT DE LA DEMANDE (Int. Cl.³) |
|---|---|---|---|
| Catégorie | Citation du document avec indication, en cas de besoin, des parties pertinentes | Revendication concernée | G 01 N 33/80 |
| X | CHEMICAL ABSTRACTS, vol. 79, no. 1, 9 juillet 1973, page 326, abrégé 3648j, COLUMBUS, OHIO (US) H. SCHULTZE-MOSGAU et al.: "Detection of specific blood-group-receptors in the placenta with agglutinins from plants (lectins) and snails (protectins)" & Z. IMMUNITAETSFORSCH., EXP. KLIN. IMMUNOL. 1972, 144(5), 482-90  * En entier * | 1-3, 8,8, 10 | |
| | CHEMICAL ABSTRACTS, vol. 80, no. 25, 24 juin 1974, page 287, abrégé 144189t, COLUMBUS, OHIO (US) F. REYES et al.: "Human normoblast A antigen seen by immunoelectron microscopy" & Nature, 1974, 247, 461-2  * En entier * | 1 | DOMAINES TECHNIQUES RECHERCHES (Int Cl.³)  G 01 N 33/80 G 01 N 33/54 |
| | CHEMICAL ABSTRACTS, vol. 80, no. 7, 18 février 1974, page 65, abrégé 34197m, COLUMBUS, OHIO (US) B. BOETTCHER et al.: "ABO blood grouping of human hair using radioactively-labeled antibodies" & Vox Sang, 1973, 25(5), 420-5  * En entier * | 1,2 | |
| | CHEMICAL ABSTRACTS, vol. 80, no. 17, 29 avril 1974, page 262, abrégé 94034r, COLUMBUS, OHIO (US) F. REYES et al.: "Detection of the erythrocyte antigen A by electron microscopy"  ./.. | 1 | CATEGORIE DES DOCUMENTS CITES  X: particulièrement pertinent A: arrière-plan technologique O: divulgation non-écrite P: document intercalaire T: théorie ou principe à la base de l'invention E: demande faisant interference D: document cité dans la demande L: document cité pour d'autres raisons  & membre de la même famille document correspondant |

Le present rapport de recherche a ete établi pour toutes les revendications

| Lieu de la recherche | Date d'achevement de la recherche | Examinateur |
|---|---|---|
| La Haye | 17.07.1981 | MEYLAERTS |

OEB Form 1503.1 06.78

**Office européen
des brevets**

**RAPPORT DE RECHERCHE EUROPEENNE**

| Catégorie | Citation du document avec indication, en cas de besoin, des parties pertinentes | Revendication concernée | CLASSEMENT DE LA DEMANDE (Int. Cl. 3) |
|---|---|---|---|
| | **DOCUMENTS CONSIDERES COMME PERTINENTS** | | |
| | & C.R. Acad. Sci., Ser. D, 1973, 277(17), 1892-32 <br><br> * En entier * <br><br>--- | | |
| A | CHEMICAL ABSTRACTS, vol. 90, no. 15, 9 avril 1979, page 466, abrégé 119465d, <br> COLUMBUS, OHIO (US) <br> R. MAGOUS et al.: "Immunosorbent method for the detection of A.B.O. blood group specificity on CEA preparations" <br><br> * En entier * <br><br>--- | 1 | |
| A | CHEMICAL ABSTRACTS, vol. 85, no. 13, 27 septembre 1976, page 458, abrégé 92072f, <br> COLUMBUS, OHIO (US) <br> J.P. BALI et al.: "Presence of blood group H antigen on a carcinoembryonic antigen, and its enzymic modification into blood group A and B specificities" <br> & Cancer Res. 1976, 36(7,pt.1), 2124-9 <br><br> * En entier * <br><br>--------- | 1 | **DOMAINES TECHNIQUES RECHERCHES (Int. Cl. 3)** |

OEB Form 1503.2   06.78